# EUROPÄISCHE PATENTSCHRIFT

(11) **EP 1 898 845 B1**
(45) Veröffentlichungstag und Bekanntmachung des Hinweises auf die Patenterteilung: **04.11.2009**
(21) Anmeldenummer: 06742702.1
(22) Anmeldetag: 26.04.2006
(51) Int. Cl.: A61F 2/72, A61F 2/78

(54) **SYSTEM AUS EINEM LINER UND EINER MYOELEKTRISCHEN ELEKTRODENEINHEIT**
SYSTEM CONSISTING OF A LINER UND A MYOELECTRIC ELECTRODE UNIT
SYSTEME COMPOSE D'UN MANCHON ET D'UNE UNITE D'ELECTRODE MYOELECTRIQUE

(30) Priorität: 04.05.2005 DE 102005021412
(43) Veröffentlichungstag der Anmeldung: 19.03.2008
(73) Patentinhaber: Otto Bock Healthcare Products GmbH, 1070 Wien (AT)
(72) Erfinder: DIETL, Hans, A-3003 Gablitz (AT); WILLBURGER, Horst, 1020 Wien / AT (AT); LANMUELLER Hermann c/o Vienna General Hospital Ebene 4/L, 1090 Wien (AT)
(74) Vertreter: Stornebel, Kai
(86) Internationale Anmeldenummer: PCT/EP2006/003873
(87) Internationale Veröffentlichungsnummer: WO 2006/117115

(56) Entgegenhaltungen:
- WO-A-02/49534
- US-A- 5 443 525
- US-A- 5 571 208

## Beschreibung

Die Erfindung betrifft ein System aus einem zwischen einem Amputationsstumpf und einem Prothesenschaft angeordneten Liner und einer myoelektrischen Elektrodeneinheit zur Erfassung myoelektrischer Signale zur Steuerung von Prothesen an einem Amputationsstumpf. Die Merkmale des Oberbegriffs des Anspruchs 1 sind aus der Druckschrift US-A-5 443 525 bekannt.

Eine Myoelektrode dient zur Erfassung und Auswertung eines Oberflächenmyogramms, auf dessen Grundlage motorisch betrieben Elemente einer Prothese gesteuert werden. Die gesamte Funktionalität einer solchen Prothese, insbesondere Armprothese, ist dabei unmittelbar von der Qualität der Myoelektrode abhängig. Aus dem Stand der Technik sind aktive Myoelektroden bekannt, die aus Ableiterelektroden zur Ableitung des Elektromyogramms sowie aus signalverstärkenden und sig-nalverarbeitenden Elementen bestehen.

Die bisher in einem geschlossenen Kunststoffgehäuse gefertigten Myoelektroden bestehen aus einer zum Amputationsstumpf ausgerichteten Eingangsschnittstelle und einer zur Prothese ausgerichteten Ausgangsschnittstelle. Die Eingangsschnittstelle wird von den auf der Oberfläche des Gehäuses gelegenen, metallischen Ableiterelektroden gebildet. Die Ausgangsschnittstelle stellt der Prothese das verstärkte und aufbereitete Elektromyogrammsignal in analoger oder digitaler Form zur Verfügung.

In den letzten Jahren ist in der prothetischen Versorgungstechnik die Entwicklung zu einer sogenannten Liner-Schafttechnik zu erkennen. Dabei wird von dem Patienten, anders als bei herkömmlichen Prothesenschäften, zunächst ein weicher, strumpfartiger Teil über den Amputationsstumpf gezogen, der als Liner bezeichnet wird. Der Patient legt im Anschluss auf den übergezogenen Liner die Prothese an. Der Liner bildet zwischen dem Amputationsstumpf und dem Prothesenschaft eine Verbindung, die einerseits den Sitz des Prothesenschaftes an dem Amputationsstumpf verbessert und andererseits den Tragekomfort der Prothese erhöht. Der Liner wird aus Materialien wie Silikon oder Polyurethan gefertigt und besitzt exzellente Hafteigenschaften auf dem Amputationsstumpf, die zusätzlich durch den luftdichten Abschluss des Liners an dem Amputationsstumpf noch wesentlich verbessert werden.

Derzeit bekannte, kabelgebundene Myoelektroden sind zusammen mit der Linertechnik nicht einsetzbar. Da diese Myoelektroden elastisch in dem Schaft aufgehängt sind, müsste in den Liner ein Fenster geschnitten werden, um den Kontakt zwischen den Ableiterelektroden und der Haut an dem Amputationsstumpf herzustellen. Dies würde zu einer Minderung der Hafteigenschaften zwischen dem Liner und dem Amputationsstumpf führen. Darüber hinaus müsste der Liner außerordentlich exakt an dem Amputationsstumpf angelegt werden, damit das darin vorhandene Fenster exakt zu der Position der Myoelektrode in dem Prothesenschaft ausgebildet ist.

Aufgabe der Erfindung ist es, ein System bereitzustellen, mit dem die aus dem Stand der Technik bekannten Nachteile vermieden werden. Erfindungsgemäß wird dieses durch ein System mit den Merkmalen des Anspruchs 1 gelöst, bei dem eine Messeinheit, die auf der dem Amputationsstumpf zugewandten Seite des Liners angeordnet ist, und eine Empfangseinheit, die auf der dem Amputationsstumpf abgewandten Seite des Liners angeordnet ist, vorgesehen sind, wobei die Messeinheit einen Sender zur kabellosen Signalübertragung der myoelektrischen Signale an die Empfangseinheit aufweist. Somit ist es möglich, einerseits die Vorteile der Liner-Technik im Bezug auf Tragekomfort und Hafteigenschaft zu nutzen und andererseits eine Prothese mit myoelektrischer Steuerung einzusetzen. Es liegt also eine telemetrische Myoelektrode vor, die aus zwei Einheiten besteht, von der eine Einheit innerhalb des Liners, der Haut des Amputationsstumpfes zugewandt, angebracht ist, und deren Empfangseinheit zwischen dem Liner und dem Prothesenschaft, bevorzugt an dem Prothesenschaft montiert ist.

Die Erfindung sieht auch vor, dass in der Messeinheit ein Verstärker zur Verstärkung der myoelektrischen Signale, ein Analog-Digital-Wandler zur Digitalisierung der Signale sowie eine Codierungseinheit zur Codierung der Signale angeordnet ist, um das verstärkte, digitalisierte und codierte Signal über eine Sendeeinheit kabellos zu der außerhalb des Liners angeordneten Empfangseinheit zu übertragen. Die Empfangseinheit leitet das empfangene Elektromyogrammsignal zur weiteren Verarbeitung innerhalb der Prothese weiter. Die Messeinheit kann auf der Innenseite des Liners fest montiert sein, beispielsweise angeklebt oder eingeschweißt, um ein problemloses Anlegen des Liners zu ermöglichen. Bevorzugt ist die Messeinheit als eine wasserdichte Kapsel in einer flachen Bauweise direkt in dem Liner integriert. Die telemetrisch myoelektrische Methode gestattet damit die Verwendung eines geschlossenen Liners innerhalb des Prothesenschaftes, der die mechatronischen Komponenten der Prothese von dem Amputationsstumpf trennt.

Um die Messeinheit verschleiß- und wartungsfrei auszubilden, ist in ihr eine Induktionsspule bzw. eine Einrichtung zur Umwandlung elektromagnetischer Wechselfelder angeordnet, so dass auf eigene Energiequellen, wie Akkumulatoren verzichtet werden kann. Die Energieübertragung erfolgt dann über ein elektromagnetisches Wechselfeld, das in der Empfangseinheit generiert wird, so dass eine telemetrische Energieübertragung durchgeführt wird.

Neben einer Übertragung der myoelektrischen Signale durch elektromagnetische Wellen ist es vorgesehen, dass der Sender optisch ausgebildet und der Empfänger in der Empfängereinheit ein optischer Sensor ist, um eine optische Signalübertragung zu ermöglichen. In der Messeinheit kann eine wirkungsgradstarke Leuchtdiode eingebaut sein, die das digitalisierte, binäre Signal der Ableitelektroden durch eine Ein-/Aus-Modulation der Leuchtstärke an die jenseits des Liners angeordnete Empfangseinheit sendet.

Die Signalübertragung kann auch per frequenzmodulierter Lastmodulation erfolgen, dabei ist die Übertragung der Stromversorgung mit der Übertragung des Elektro-myogrammsignals gekoppelt. Die Messeinheit verstimmt dabei den Resonanzkreis, der zur Übertragung der Versorgungsenergie verwendet wird. Die Verstimmung ist moduliert mit dem binären Signalstrom. Diese Verstimmung wird in der Empfangseinheit detektiert und demoduliert.

Die Signalübertragung kann auch per amplitudenmodulierter Lastmodulation erfolgen, dabei ist die Übertragung der Stromversorgung mit der Übertragung des Elektromyogrammsignals gekoppelt. Die Messeinheit dämpft dabei den Resonanzkreis, der zur Übertragung der Versorgungsenergie verwendet wird. Die Dämpfung ist moduliert mit dem binären Signalstrom. Diese Dämpfung wird in der Empfangseinheit detektiert und demoduliert.

Auch ist es vorgesehen, die Signalübertragung per amplitudenmoduliertem Träger durchzuführen. Dabei wird ein im Sender erzeugter Träger mit dem binären Datenstrom moduliert. Der Träger wird über eine eigene, von der Energieversorgung unabhängige elektromagnetische Kopplung an die Empfangseinheit gesendet und dort demoduliert.

Nachfolgen wird ein Ausführungsbeispiel der Erfindung anhand der beigefügten Figuren näher erläutert.
- Figur 1 -: zeigt die Zuordnung von Amputationsschaft, Liner und Prothese;
- Figur 2-: zeigt eine schematische Anordnung der Elektrodeneinheit an dem Amputationsstumpf und den Liner; sowie
- Figur 3 -: eine detaillierte Darstellung der funktionalen Einheiten der Elektrodeneinheit.

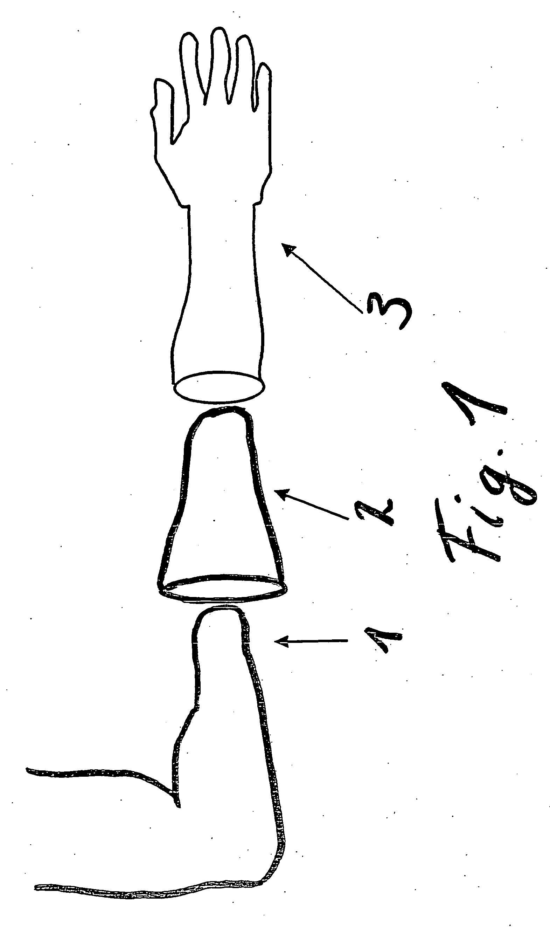

In der Figur 1 ist die grundsätzliche Anordnung einer Prothese 3 an einem Amputationsstumpf 1 gezeigt, bei der zwischen dem Amputationsstumpf 1 und dem Prothesenschaft 3 ein Liner 2 angeordnet ist. Der Liner 2, der aus Silikon, Polyurethan oder anderen, vorzugsweise an dem Amputationsstumpf haftenden und schützenden Materialien gefertigt ist, wird individuell an dem Amputationsstumpf 1 angepasst und vor Anlegen des Prothesenschaftes 3 angezogen. Der Liner 2 ist relativ weich und bildet eine Verbindungsschicht zwischen der Haut des Amputationsstumpfes 1 und der inneren Auskleidung des Prothesenschaftes 3.

Um eine Steuerung beweglicher Komponenten des Prothesenschaftes 3, beispielsweise im Fingerbereich, zu ermöglichen, werden über eine nicht dargestellte Myoelektrode Signale von Muskelaktivitäten aufgenommen. Das sogenannte Ober-flächenmyogramm wird aufgenommen und nach Bearbeitung der Signale dienen diese Signale zur Steuerung der mechatronischen Komponenten.

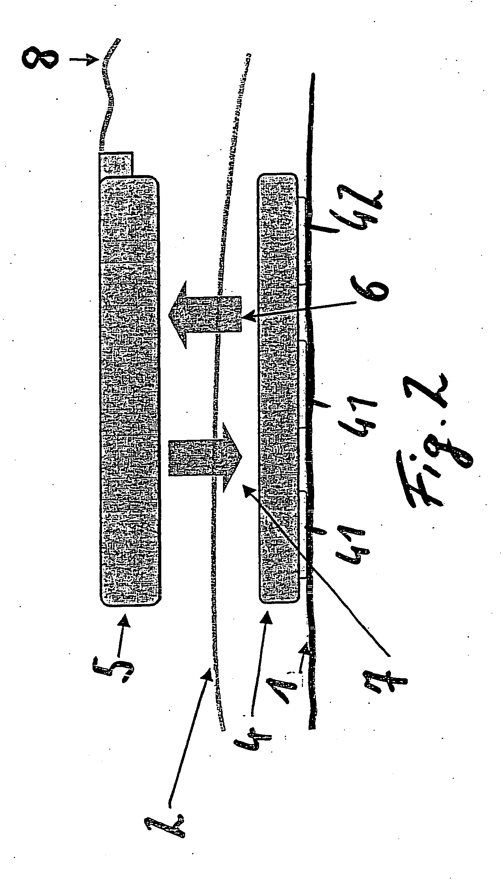

In der Figur 2 ist im Querschnitt eine schematische Anordnung einer Elektrodeneinheit gezeigt, die aus einer Messeinheit 4 und einer Empfängereinheit 5 aufgebaut ist. Die Messeinheit 4 ist zwischen den Oberflächen des Amputationsstumpfes 1 und dem Liner 2 auf der Hautoberfläche des Amputationsstumpfes 1 angeordnet. Ableiterelektroden 41 und eine Erdungselektrode 42 liegen unmittelbar auf der Hautoberfläche des Amputationsstumpfes 1 auf und erfassen myoelektrische Signale. Diese Signale werden in der Messeinheit 4 aufbereitet und kabellos über eine Sendeeinheit durch den Liner 2 zu einer jenseits des Liners 2 angeordneten Empfängereinheit 5 geleitet. Die Signalübertragung 6 erfolgt kabellos über optische Signale oder alternativ über Amplitudenmodulation elektromagnetischer Signale.

Um die Messeinheit 4 mit Energie zu versorgen, wird ein elektromagnetisches Wechselfeld 7 auf die Messeinheit 4 gerichtet, wo beispielsweise über eine Induktionsspule Strom induziert wird.

Die Signalübertragung 6 kann auch per frequenzmodulierter oder amplitudenmodulierter Lastmodulation erfolgen. Dabei ist die Übertragung der Stromversorgung 7 mit der Übertragung 6 des Elektromyogrammsignals gekoppelt. Die Messeinheit 4 verstimmt dabei einen Resonanzkreis, der zur Übertragung 7 der Versorgungsenergie für die Messeinheit 4 verwendet wird. Die Verstimmung ist mit dem binären Signalstrom moduliert und kann von der Empfangseinheit 5 detektiert und demoduliert werden. Alternativ kann die Signalübertragung 6 auch über eine amplitudenmodulierte Lastmodulation erfolgen.

In der Empfängereinheit 5 sind Einrichtungen zur Signalverarbeitung und Empfänger angeordnet, die die empfangenen Signale aufbereiten und bevorzugt per Kabel 8 an die mechatronischen Komponenten der Prothese 3 weiterleiten. Die Empfängereinheit 5 ist bevorzugt auf der Innenseite des Prothesenschaftes 3 angeordnet.

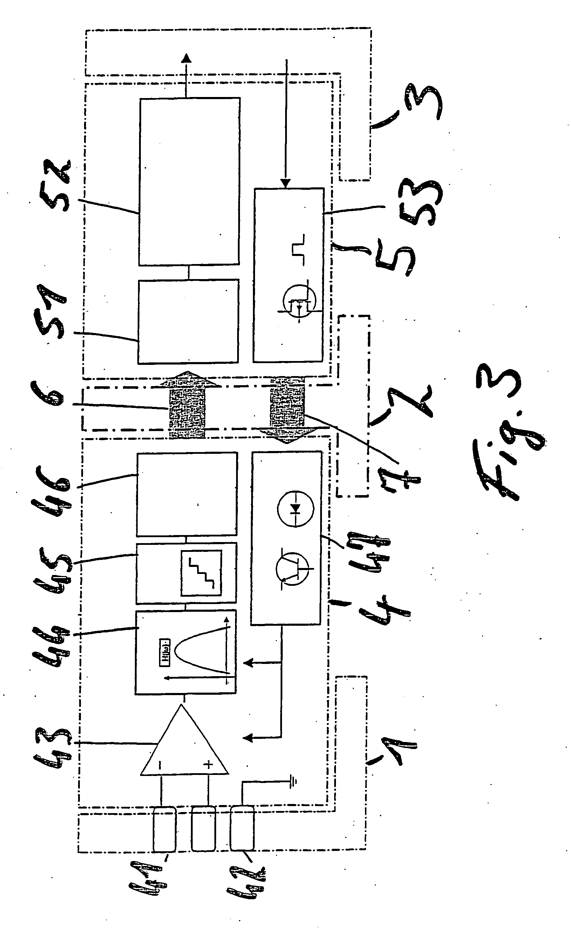

In der Figur 3 ist in detaillierter Art und Weise der Aufbau der Messeinheit 4 und der Empfangseinheit 5 gezeigt. Die Ableiterelektroden 41 und die Erdungselektrode 42 sind unmittelbar dem Amputationsstumpf 1 zugeordnet. Von den Ableiterelektroden 41 wird das Signal über einen Operationsverstärker 43 einem Filter 44 zugeführt, von dem es dann über einen Analog-Digital-Wandler 45 zu einer Codier-, Modulier- und Sendereinheit 46 geleitet wird. Diese Codier-, Modulier- und Sendereinheit 46 überträgt telemetrisch einen Signalstrom 6 durch den Liner 2 zu einem Empfängermodul 51, in dem das Signal demoduliert und decodiert wird. Von dem Empfängermodul 51 wird das demodulierte, decodierte Signal zur weiteren Signalverarbeitung einer entsprechenden Signalverarbeitungseinheit 52 zugeführt, von der es aus über ein nicht dargestelltes Kabel zu Elementen innerhalb des Prothesenschaftes 3 geleitet wird.

Die Empfangseinheit 5 ist auf der Innenseite des Prothesenschaftes 3 befestigt, während die Messeinheit 4 auf der Innenseite des Liners 2 befestigt ist. Vorzugsweise sind die Messeinheit 4 und die Empfangseinheit 5 so angeordnet, das sie zueinander ausgerichtet sind und einander gegenüberliegen. Dadurch ist eine gute telemetrische Signalübertragung gewährleistet. Die Signalübertragung 6 kann sowohl optisch als auch elektromagnetisch erfolgen.

Um den Filter 44, den Analog-Digital-Wandler 45, die Codiereinheit und den Sender 46 sowie den Operationsverstärker 43 mit Energie zu versorgen, ist eine Stromversorgungseinheit 47 mit einem Gleichrichter aufgebaut, in der von einer Wechselrichtereinheit 53 ausgesendete elektromagnetische Wechselfelder umgewandelt werden. Dabei wird eine elektromagnetische Kopplung zwischen der Messeinheit 4 und der Empfangseinheit 5 gebildet, die zur Übertragung 7 der Versorgungsenergie verwendet wird.

## Patentansprüche

1. System aus einem zwischen einem Amputationsstumpf (1) und einem Prothesenschaft (3) angeordneten Liner (2) und einer myoelektrischen Elektrodeneinheit (4, 5) zur Erfassung myoelektrischer Signale, bei der die Elektrodeneinheit (4, 5) eine Messeinheit (4), die auf der den Amputationsstumpf (1) zugewandten Seite des Liners (2) angeordnet ist, und eine Empfangseinheit (5), die auf der dem Amputationsstumpf (1) abgewandten Seite des Liners (2) angeordnet ist, aufweist, daduch **gekennzeichnet**, daß die Messeinheit (4) einen Sender (46) zur kabellosen Signalübertragung der myoelektrischen Signale an einen Empfänger (51) in der Empfangseinheit (5) aufweist.

2. System nach Anspruch 1, **dadurch gekennzeichnet, dass** in der Messeinheit (4) ein Verstärker (43) zur Verstärkung der myoelektrischen Signale angeordnet ist.

3. System nach Anspruch 1 oder 2, **dadurch gekennzeichnet, dass** in der Messeinheit (4) ein Analog-Digital-Wandler (45) zur Digitalisierung des myoelektrischen Signals angeordnet ist.

4. System nach einem der voranstehenden Ansprüche, **dadurch gekennzeichnet, dass** in der Messeinheit (4) eine Codiereinheit (46) zur Codierung der zu sendenden Signale angeordnet ist.

5. System nach einem der voranstehenden Ansprüche, **dadurch gekennzeichnet, dass** in der Messeinheit (4) eine Induktionsspule und eine Gleichrichtereinheit (47) zur Energieversorgung der Messeinheit (4) angeordnet ist.

6. System nach einem der voranstehenden Ansprüche, **dadurch gekennzeichnet, dass** die Messeinheit (4) an dem Liner (2) befestigt ist.

7. System nach einem der voranstehenden Ansprüche, **dadurch gekennzeichnet, dass** die Messeinheit (4) wasserdicht abgekapselt ist.

8. System nach einem der voranstehenden Ansprüche, **dadurch gekennzeichnet, dass** die Empfangseinheit (5) eine Einrichtung (53) zur Erzeugung elektromagnetischer Wechselfelder und eine Induktionsspule zur Energieübertragung aufweist.

9. System nach einem der voranstehenden Ansprüche, **dadurch gekennzeichnet, dass** die Empfangseinheit (5) an dem Prothesenschaft (3) befestigt ist.

10. System nach einem der voranstehenden Ansprüche, **dadurch gekennzeichnet, dass** der Sender (46) als ein optischer Sender ausgebildet ist und dass in der Empfangseinheit (5) ein optischer Sensor (51) als Empfänger angeordnet ist.

11. System nach einem der Ansprüche 1 bis 9, **dadurch gekennzeichnet, dass** die Messeinheit (4) und die Empfangseinheit (5) einen Schwingkreis ausbilden und die Signalübertragung (6) über eine frequenz- oder amplitudenmodulierte Lastmodulation erfolgt.

12. System nach einem der Ansprüche 1 bis 9, **dadurch gekennzeichnet, dass** die Signalübertragung (6) durch Amplitudenmodulation eines im Sender (46) erzeugten Trägersignals erfolgt, das der Empfänger (51) entsprechend demoduliert.

## Claims

1. System consisting of a liner (2) arranged between an amputation stump (1) and a prosthesis shaft (3) and a myoelectrical electrode unit (4, 5) for recording of myoelectrical signals, for which the electrode unit figure 5 has a measurement unit (4) which is located on that side of the liner (2) which is directed to the amputation stump (1) and a receiver unit (5) which is located on that side of the liner (2) which is directed away from the amputation stump (1), **characterized in that** the measurement unit (4) has a sender (46) for wireless signal transmission of the myoelectrical signals to an receiver (51) in the receiver unit (5).

2. System according to claim 1, **characterized in that** there is an amplifier (43) in the measurement unit (4) for amplification of the myoelectrical signals.

3. System according to claims 1 or 2, **characterized in that** in the measurement unit (4) has an analog-digital converter (45) for digitizing of the myoelectrical signals.

4. System according to one of the previous claims, **characterized in that** there is a coding unit (46) in the measurement unit (4) for coding the signals to be sent.

5. System according to one of the previous claims, **characterized in that** there is an induction coil and a rectifier (47) in the measurement unit (4) for power provision of the measurement (4)

6. System according to one of the previous claims, **characterized in that** the measurement unit (4) is attached to the liner (2).

7. System according to one of the previous claims, **characterized in that** the measurement unit (4) is in a watertight encapsulation.

8. System according to one of the previous claims, **characterized in that** the receiver unit (5) has a unit (53) for creation of electromagnetic alternating fields and an induction coil for energy transmission.

9. System according to one of the previous claims, **characterized in that** the receiver unit (5) is attached to the prosthesis shaft (3).

10. System according to one of the previous claims, **characterized in that** the sender (46) is an optical sender and that there is an optical sensor (51) in the receiver unit (5) as a receiver.

11. System according to one of the claims 1 to 9, **characterized in that** the measurement unit (4) and the receiver unit (5) create a resonant circuit and the signal transmission (6) occurs via a frequency of amplitude modulated load modulation.

12. System according to one of the previous claims 1 to 9, **characterized in that** the signal transmission (6) occurs through amplitude modulation of a carrier signal created in the sender (46), the carrier signal is demodulated by the receiver (51) accordingly.

## Revendications

1. Système composé d'un liner (2) agencé entre un moignon d'amputation (1) et un fût de prothèse (3), et d'une unité d'électrode (4, 5) myoélectrique pour l'acquisition de signaux myoélectriques, dans laquelle l'unité d'électrode (4, 5) présente une unité de mesure (4) qui est agencée du côté du liner (2) tourné vers le moignon d'amputation (1), et une unité de réception (5) qui est agencée du côté du liner (2) opposé au moignon d'amputation (1), **caractérisé en ce que** l'unité de mesure (4) présente un émetteur (46) pour la transmission sans fil des signaux myoélectriques à un récepteur (51) dans l'unité de réception (5).

2. Système selon la revendication 1, **caractérisé en ce qu'**un amplificateur (43) est agencé dans l'unité de mesure (4) pour amplifier les signaux myoélectriques.

3. Système selon la revendication 1 ou 2, **caractérisé en ce qu'**un convertisseur analogique-numérique (45) est agencé dans l'unité de mesure (4) pour numériser le signal myoélectrique.

4. Système selon l'une des revendications précédentes, **caractérisé en ce qu'**une unité de codage (46) est agencée dans l'unité de mesure pour coder les signaux à envoyer.

5. Système selon l'une des revendications précédentes, **caractérisé en ce qu'**une bobine d'induction et une unité de redressement (47) sont agencées dans l'unité de mesure pour alimenter en énergie l'unité de mesure (4).

6. Système selon l'une des revendications précédentes, **caractérisé en ce que** l'unité de mesure (4) est fixée au liner (2).

7. Système selon l'une des revendications précédentes, **caractérisé en ce que** l'unité de mesure (4) est encapsulée de façon étanche à l'eau.

8. Système selon l'une des revendications précédentes, **caractérisé en ce que** l'unité de réception (5) présente un dispositif (53) pour produire des champs alternatifs électromagnétiques et une bobine d'induction pour la transmission d'énergie.

9. Système selon l'une des revendications précédentes, **caractérisé en ce que** l'unité de réception (5) est fixée au fût de prothèse (3).

10. Système selon l'une des revendications précédentes, **caractérisé en ce que** l'émetteur (46) est réalisé sous la forme d'un émetteur optique, et **en ce qu'**un capteur optique (51) est agencé en tant que récepteur dans l'unité de réception (5).

11. Système selon l'une des revendications 1 à 9, **caractérisé en ce que** l'unité de mesure (4) et l'unité de réception (5) forment un circuit oscillant et la transmission de signal (6) s'effectue par l'intermédiaire d'une modulation de charge modulée en fréquence ou en amplitude.

12. Système selon l'une des revendications 1 à 9, **caractérisé en ce que** la transmission de signal (6) s'effectue par modulation d'amplitude d'un signal porteur produit dans l'émetteur (46), signal que le récepteur (51) démodule en conséquence.
